# EUROPEAN PATENT APPLICATION

(11) **EP 3 315 605 A1**
(43) Date of publication of application: **02.05.2018**
(21) Application number: 16814025.9
(22) Date of filing: 11.04.2016
(51) Int. Cl.: C12N 15/00, C12N 15/09, C12Q 1/68

(54) **PRIMER SET FOR DETECTING 1,4-DIOXANE DEGRADING BACTERIUM, AND METHOD FOR DETECTING AND QUANTIFYING 1,4-DIOXANE DEGRADING BACTERIUM**

(30) Priority: 24.06.2015 JP 2015126202
(71) Applicant: Taisei Corporation, Tokyo 163-0606 (JP); Osaka University, Suita-shi Osaka 565-0871 (JP); School Juridical Person Kitasato Institute, Tokyo 108-8641 (JP)
(72) Inventor: YAMAMOTO, Norifumi, Tokyo 163-0606 (JP); SAITO, Yuji, Tokyo 163-0606 (JP); TAKI, Hironori, Tokyo 163-0606 (JP); IKE, Michihiko, Suita-shi Osaka 565-0871 (JP); KURODA, Masashi, Suita-shi Osaka 565-0871 (JP); SEI, Kazunari, Sagamihara-shi Kanagawa 252-0373 (JP); INOUE, Daisuke, Sagamihara-shi Kanagawa 252-0373 (JP)
(74) Representative: Grünecker Patent- und Rechtsanwälte PartG mbB
(86) International application number: PCT/JP2016/061655
(87) International publication number: WO 2016/208254

(57) **Abstract**

An object of the present invention is to provide a primer that allows for detection and quantification of 1,4-dioxane-degrading bacteria in a quick, highly accurate manner. To achieve the object, a primer set is provided which comprises a primer that contains a base sequence set forth by SEQ ID NO: 1 and a primer that contains a base sequence set forth by SEQ ID NO: 2.

## Description

### Technical Field

The present invention relates to a primer set used for polymerase chain reaction (hereinafter referred to as "PCR"), as well as a method for detecting and quantifying 1,4-dioxane-degrading bacteria utilizing this primer set.

### Background Art

1,4-dioxane is a cyclic ether expressed by the following formula (1). 1,4-dioxane is excellent in compatibility with water or organic solvent and is usually used as a reaction solvent for organic synthesis.

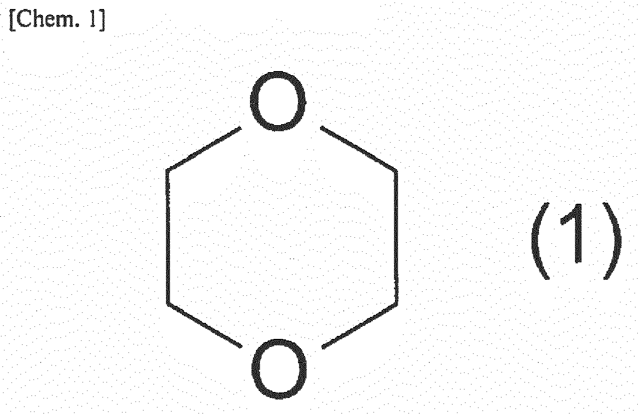

The manufacturing and import volume of 1,4-dioxane in Japan in 2010 was about 4500 t/year and it is presumed that 1,4-dioxane is released into the environment by about 300 t/year. 1,4-dioxane is water-soluble, and thus 1,4-dioxane diffuses over a wide area when it is released into a water environment. Also, 1,4-dioxane is inferior in volatility, adsorption to solids, photodegradability, hydrolyzability, and biodegradability, and thus it is difficult to be removed from water. Since 1,4-dioxane has acute toxicity and chronic toxicity, and further, carcinogenicity is pointed out, the contamination of water environments by 1,4-dioxane is considered to adversely affect humans and animals and plants. Therefore, in Japan, 1,4-dioxane is regulated by a tap water quality standard (0.05 mg /L or less), an environmental standard (0.05 mg /L or less), and a wastewater standard (0.5 mg/ L or less).

It is not possible to sufficiently remove 1,4-dioxane from water by conventional treatment methods such as the activated sludge method and the activated carbon adsorption method. The effectiveness of 1,4-dioxane treatment is confirmed only in the accelerated oxidation method using a plurality of physicochemical oxidation methods such as an ozone treatment with addition of hydrogen peroxide (O3/H₂O₂), an ozone treatment under ultraviolet irradiation (O₃/UV), an ozone treatment under irradiation with radiation or ultrasonic wave in combination. However, the accelerated oxidation method is not widely used due to the high initial cost and running cost. Furthermore, in Non-Patent Literature 1, it is reported that the efficiency of 1,4-dioxane treatment by the accelerated oxidation method decreases when an organic substance other than 1,4-dioxane is present.

There is a demand for a method for treating water containing 1,4-dioxane stably at a low cost, and a biological treatment by 1,4-dioxane-degrading bacteria is proposed in Patent Literature 1 and Non-Patent Literature 2. The biological treatment provides a method whereby 1,4-dioxane-degrading bacteria are introduced to an aeration tank, soil, underground water, etc., to decompose the 1,4-dioxane contained therein. However, the amount of bacterial cells of 1,4-dioxane-degrading bacteria changes daily. Since a decrease in 1,4-dioxane-degrading bacteria means a drop in treatment capability, it is necessary to monitor the amount of bacterial cells of 1,4-dioxane-degrading bacteria and add 1,4-dioxane-degrading bacteria if the amount of bacterial cells is low. One method to monitor the amount of bacterial cells is to culture a sample containing 1,4-dioxane-degrading bacteria in an agar plate medium and visually count the number of colonies several days later. However, this method presents problems such as that culturing takes a long time, and that it is not clear whether the formed colonies represent 1,4-dioxane-degrading bacteria or other bacteria.

1,4-dioxane-degrading bacteria are largely classified into two types: bacteria that decompose and assimilate 1,4-dioxane as a single carbon source, and bacteria that decompose 1,4-dioxane through co-metabolic reaction in the presence of tetrahydrofuran or other components. In Non-Patent Literatures 3 and 4, it is reported that the THF monooxygenases possessed by these 1,4-dioxane-degrading bacteria are involved in the decomposition of 1,4-dioxane. THF monooxygenase is classified as one type of soluble iron (II) monooxygenase (SDIMO) which is responsible for the initial oxidation of various hydrocarbons. Also, methane/propane monooxygenase and the like are contained in SDIMO (Non-Patent Literature 5). Furthermore, in Non-Patent Literature 6, it is reported that bacteria having SDIMO other than THF monooxygenase may also decompose 1,4-dioxane.

### Background Art Literature

### Patent Literature

Patent Literature 1: Japanese Unexamined Patent Application Publication No. 2008-306939
Non-patent Literature 1: K. KOSAKA, H. YAMADA, S. MATSUI, and K. SHISHIDA: The effects of the co-existing compounds on the decomposition of micropollutants using the ozone/hydrogen peroxide process. Water Sci. Technol., 42, pp.353-361, 2000.
Non-Patent Literature 2: KAZUNARI SEI, MICHIHIKO IKE: Challenge for biotreatment of groundwater contaminated with 1,4-dioxane by 1,4-dioxane-degrading bacteria, J. Water and Wastewater, Vol.53, No.7, 2011.
Non-Patent Literature 3: H. MASUDA, K. McCLAY, R. J. STEFFAN, and G. J. ZYLSTRA: Biodegradation of tetrahydrofuran and 1,4-dioxane by soluble diiron monooxygenase in Pseudonocardia sp. strain ENV478. J. Mol. Microbiol. Biotechnol. 22(5), pp. 312-316, 2012.
Non-Patent Literature 4: A. GROSTERN, C. M. SALES, W.-Q. ZHUANG, O. ERBILGIN, and L. ALVAREZ-COHEN: Glyoxylate metabolism is a key feature of the metabolic degradation of 1,4-dioxane by Pseudonocardia dioxanivorans strain CB1190. Appl. Environ. Microbiol., 78(9), pp. 3298-3308, 2012.
Non-Patent Literature 5: N. V. COLEMAN, N. B. BUI, and A. J. HOLMES: Soluble di-iron monooxygenase gene diversity in soils, sediments and ethane enrichments. Environ. Microbiol., 8(7), pp. 1228-1239, 2006.
Non-Patent Literature 6: S. MAHENDRA, and L. ALVAREZ-COHEN: Kinetics of 1,4-dioxane biodegradation by monooxygenase-expressing bacteria. Environ. Sci. Technol., 40(17), pp. 5435-5442, 2006.

### Summary of the Invention

### Problems to Be Solved by the Invention

An object of the present invention is to provide a primer that allows for detection and quantification of 1,4-dioxane-degrading bacteria in a quick, highly accurate manner.

### Means for Solving the Problems

1. A primer set, comprising a primer that contains a base sequence set forth by SEQ ID NO: 1 and a primer that contains a base sequence set forth by SEQ ID NO: 2.
2. A method for detecting 1,4-dioxane-degrading bacteria, comprising performing a PCR method using a primer set according to 1, and determining, based on whether or not gene amplification has occurred, whether or not microorganisms having the *thmC* gene are present.
3. A method for quantifying 1,4-dioxane-degrading bacteria, characterized by quantifying microorganisms having the *thmC* gene based on the amplified amount of *thmC* genes obtained by the real-time PCR method that uses a primer set according to 1.

### Effects of the Invention

Using the primer set proposed by the present invention, the *thmC* gene, which is a gene involved in the decomposition of 1,4-dioxane contained in 1,4-dioxane-degrading bacteria and is presumed to be coding the monooxygenase component MmoB/DmpM being a protein constituting THF monooxygenase, can be amplified specifically.

As the *thmC* gene is amplified, whether or not 1,4-dioxane-degrading bacteria are present can be determined. Also, the amount of bacterial cells of 1,4-dioxane-degrading bacteria present in a sample can be estimated accurately from the Ct value according to the real-time PCR method. The ability to accurately quantify the amount of bacterial cells of 1,4-dioxane-degrading bacteria makes it possible to add 1,4-dioxane-degrading bacteria immediately if the amount of bacterial cells is low, which helps maintain the treatment capability of 1,4-dioxane in a stable manner.

### Brief Description of the Drawings

[Fig. 1] Calibration curve of a quantitative monitoring system for strain D17.
[Fig. 2] Correlation (A) between the quantified value according to the real-time PCR method and the turbidity, and correlation (B) between the quantified value according to the real-time PCR method and the quantified value according to the direct counting method.

### Mode for Carrying Out the Invention

1,4-dioxane-degrading bacteria (hereinafter referred to as "degrading bacteria") are present in nature, and can be screened by culturing sludge, etc., that has been contaminated with 1,4-dioxane, in a medium that contains 1,4-dioxane as its only carbon source. Among the degrading bacteria, *Pseudonocardia* sp. D17, *Pseudonocardia dioxanivorans* CB1190, *Afipia* sp. D1, Mycobacterium sp. PH-06, etc., are known, for example. *Pseudonocardia* sp. D17 (hereinafter referred to as "strain D17") was internationally deposited on August 29, 2014 at the National Institute of Technology and Evaluation's Patent Microorganisms Depositary Center (NPMD) (Kazusakamatari 2-5-8, Kisarazu-city, Chiba Prefecture, Japan (Postal Code 292-0818)) as accession No. NITE BP-01927. *Pseudonocardia dioxanivorans* CB1190 (referred to as strain CB1190, hereinafter) can be purchased from the United States ATCC (ATCC 55486). In addition to ATCC in the United States, it can also be purchased at JCM (Institute of Physical and Chemical Research, Bioresource Center, Microbial Material Development Office) and DSM in Germany.

In Non-Patent Literatures 3 and 4 above, it is reported that THF monooxygenase contained in the degrading bacteria is involved in the degradation of 1,4-dioxane. The inventors of the present invention found that the degrading bacteria can be detected and quantified using the *thmC* gene which is one of the genes involved in the degradation of 1,4-dioxane contained in the degrading bacteria and which is presumed to be coding the monooxygenase component MmoB/DmpM being a protein constituting THF monooxygenase.

To be specific, 1,4-dioxane-degrading bacteria can be detected by amplifying the *thmC* gene through a PCR that uses a primer set comprising a primer which serves as a forward primer and contains a base sequence set forth by SEQ ID NO: 1 (5'-TGATTATGTGGGGCTGGTTATG-3'), and a primer which serves as a reverse primer and contains a base sequence set forth by SEQ ID NO: 2 (5'-CGAGGAAAGTTGTGTTCGTGATG-3 ').

For the PCR method, any of the MPN-PCR method, competitive PCR method, and real-time PCR method can be used; however, the real-time PCR method is preferred because it can produce results in a quick, highly accurate manner. Under the real-time PCR method, an amplified product is detected by fluorescent light. For the detection method, the intercalator method whereby a pigment emitting fluorescent light is intercalated, the fluorescent probe method whereby a fluorescent pigment is bonded, or the like, can be used without being limited in any way.

In other words, whether or not 1,4-dioxane-degrading bacteria, which are microorganisms having the *thmC* gene, are present in a sample can be determined by amplifying and detecting the *thmC* gene.

Now, the real-time PCR method provides a method for monitoring and analyzing in real time the PCR-amplified amount of DNA fragments. The real-time PCR method allows for quantitative analysis of the concentration, in a sample, of a DNA having a specific base sequence.

According to the real-time PCR method, a specific DNA is amplified, and when the amplified amount reaches the fluorescence detection limit, a sudden increase in fluorescence intensity is observed. The number of cycles after which this sudden increase in fluorescence intensity is observed is called the "threshold cycle" (hereinafter referred to as "Ct value"). Under the PCR method, the amount of DNA doubles every cycle, and the DNA is amplified exponentially. The greater the amount of DNA initially contained in the sample, the fewer the cycles that are needed to reach the fluorescence detection limit, and the smaller the CT value becomes.

There is a linear relationship between the Ct value and a common logarithm of the initial DNA amount, and a calibration curve can be drawn based on this relationship. To be specific, a calibration curve can be drawn by measuring the Ct values of multiple samples having different DNA concentrations using the real-time PCR method, and then plotting the Ct values along the vertical axis and the initial DNA amounts before the start of PCR along the horizontal axis. This calibration curve represents the relationship between the DNA concentration and Ct value of a sample, which means that, by measuring the Ct value of a sample of unknown DNA concentration using the real-time PCR method, the amount of the DNA contained in the sample can be determined.

In other words, the linear relationship that exists between the quantified amount of *thmC* genes and the amount of bacterial cells of 1,4-dioxane-degrading bacteria, allows the amount of bacterial cells of the degrading bacteria in a sample to be estimated from the amount of *thmC* genes quantified by the real-time PCR method.

The degrading bacteria are eaten by the predatory organisms positioned above them in the food chain, or grow, and thus their amount of bacterial cells fluctuates daily. In the treatment of 1,4-dioxane contamination using the degrading bacteria, the treatment capability changes in proportion to the amount of bacterial cells of degrading bacteria, and as the amount of bacterial cells decreases, the treatment capability drops. By observing the Ct value of *thmC* genes using the real-time PCR method, the amount of bacterial cells of degrading bacteria can be quantified in a quick, highly accurate manner, which means that, when the amount of bacterial cells is smaller than the amount needed to treat 1,4-dioxane, 1,4-dioxane-degrading bacteria can be added to increase the treatment capability.

Examples of the present invention are explained more specifically below; however, the present invention is not limited to these examples.

### Examples

### Example 1 Specification of a group of genes involved in the decomposition of 1,4-dioxane

Open reading frames (ORFs) were predicted from draft genome data of strain D17, from which annotated ORFs were extracted through BLAST search (BLASTP), and the eArray System by Agilent Technologies was used to design DNA probes for 7511 sequences. The 8x15K format by Agilent Technologies was used to produce custom DNA microarray slides.

Colonies of strain D17 were inoculated into an inorganic salt medium to which 500 mg/L of 1,4-dioxane had been added (1 g/L of K₂HPO₄, 1 g/L of (NRO₂SO₄, 50 mg/L of NaCl, 200 mg/L of MgSO₄•7H₂O, 10 mg/L of FeCl₃, 50 mg/L of CaCl₂, pH7.0), and cultured under rotary shaking at 28°C and 120 rpm. When the concentration of 1,4-dioxane in the culture solution was measured periodically during the culture period, the concentration of 1,4-dioxane dropped by approx. 40% after 6 days, and dropped by approx. 60% after 7 days. Accordingly, a part of the culture solution after 7 days was transferred into a new inorganic salt medium to which 1,4-dioxane had been added (concentration of 1,4-dioxane: 500 mg/L), and the culture solution thus obtained was used to conduct DNA microarray expression analysis.

RNA in the culture solution was extracted using the RNAspin Mini Kit (manufactured by GE Healthcare) and condensed by means of ethanol precipitation, after which the RiboMinus Transcriptome Isolation (Bacteria) Kit (manufactured by Life Technologies) was used to remove rRNA. To the remaining mRNA, Poly A was added using the E. coli Poly (A) Polymerase (manufactured by New England BioLabs) to synthesize a Cy3 labeled cRNA using the Low Input Quick Amp Labeling Kit (manufactured by Agilent Technologies), which was then refined using the RNeasy Mini Kit (manufactured by Qiagen). The refined Cy3 labeled cRNA was pretreated using the Gene Expression Hybridization Kit (manufactured by Agilent Technologies), and then hybridized using the aforementioned custom DNA microarray slides. After 16 hours of hybridization in a hybridization oven (65°C, 10 rpm), the custom DNA microarray slides were washed using the Gene Expression Wash Buffer (manufactured by Agilent Technologies) and scanned using the GenePix 4000B (manufactured by Molecular Devices), and the fluorescent intensity of each probe was obtained as a numerical value using the GenePix Pro 7 Software (manufactured by Molecular Devices). As a result of microarray expression analysis, those probes (genes) with a signal-to-noise (S/N) ratio of 150 or more were defined as strong expression genes and used for data analysis.

Of the 7511 probes installed on the microarrays, 285 probes with an S/N ratio of 150 or more were determined to have expressed strongly. Of these 285 probes, 175 probes were function-estimated as a result of BLASTP search, so by using the underlying 175 genes as targets, estimation of the 1,4-dioxane-degradation mechanism was attempted. When the results were compared against the published results of a total genome analysis and an estimation of a group of genes involved in the decomposition of 1,4-dioxane of strain CB1190 which is a 1,4-dioxane-degrading bacterium, 152 out of the 175 genes were also present in the CB1190 strain. Table 1 shows, among the probes that expressed strongly in the decomposition of 1,4-dioxane using strain D17, those included in the genes that would express strongly in the decomposition of 1,4-dioxane using strain CB1190 as described in Non-Patent Literature 4 above. In Non-Patent Literature 4 above, it is reported that, with strain CB1190, the thm genes are responsible for the initial oxidation reaction through which 1,4-dioxane changes to 2-hydroxy-1,4-dioxane and 2-hydroxy-ethoxy acetaldehyde.

**[Tabl 1]**

| | | | |
|---|---|---|---|
| 0368 | 2214 | Transglycosylase associated protein | |
| 6516 | 1672 | Short-chain dehydrogenase/reductase SDR | |
| 2755 | 682 | Transcription factor WhiB | |
| 0820 | 504 | NLP/P60 protein | |
| 5968 | 498 | IstB domain-containing protein ATP-hinding protein | |
| 7537 | 468 | Nitrilotriacetate monooxygenase FMN-dependent oxidoreductase | |
| 0864 | 372 | Monooxygenase component MmoB/DmpM | *thmC* |
| 0863 | 286 | Methane/phenol/toluene hydroxylase | *thmB* |
| 5884 | 276 | Ferredoxin--NAD(+) reductase | *thmD* |
| 0859 | 218 | Ethyl tert-butyl ether degradation EthD | |
| 4108 | 212 | Amidase | |
| 0860 | 194 | Betaine-aldehyde dehydrogenase | |
| 0862 | 186 | Ferredoxin--NAD(+) reductase | *thmD* |
| 0861 | 184 | Soluble di-iron monooxygenase alpha subunit | *thmA* |
| 7547 | 178 | Regulatory protein ArsR | |
| 5815 | 168 | D-lactate dehydrogenase | |
| 5650 | 156 | Short-chain dehydrogenase/reductase SDR | |
| 2857 | 152 | Hydroxyacid-oxoacid transhydrogenase | |
| 6262 | 152 | Methane/phenol/toluene hydroxylase | *thmB* |

Strain D17 has two thmA genes (coding enzymes: soluble di-iron monooxygenase alpha subunit), seven thmD genes (coding enzyme: ferredoxin-NAD (+) reductase), four thmB genes (coding enzyme: methane/phenol/toluene hydroxylase), and four *thmC* genes (coding enzyme: monooxygenase component MmoB/DmpM) (Probe IDs 0864, 3016, 5490, 6263), on its genome, and from their positions in the genome, two sets of the above form a cluster (thmADBC; Probe IDs 0861 to 0864 and 6260 to 6263). Of these genes, those of Probe IDs 0861 to 0864 were all observed to have expressed strongly, as shown in Table 1, which confirms that the corresponding thm cluster expresses strongly when 1,4-dioxane is decomposed. In other words, it is suggested that, also with strain D17, the thm cluster is involved in the initial oxidation of 1,4-dioxane, as with strain CB1190.

### Example 2 Quantification of the amount of bacterial cells based on the real-time PCR method targeting thm genes

From draft genome data of strain D17, the base sequences of thm genes were specified and the Primer3 Program (T. KORESSAAR, M. REMM: Bioinformatics, 23, 10, 1289-1291 (2007) "Enhancements and modifications of primer design program Primer3," A. UNTERGASSER, I. CUTCUTACHE, T. KORESSAAR, J. YE, B.C. FAIRCLOTH, M. REMM, S.G. ROZEN: Nucleic Acids Res. 40, 15, e115 (2012) "Primer3 - new capabilities and interfaces") was used to extract candidate primers for real-time PCR. Next, the Primer-Blast Program (J. YE, G. COULOURIS, 1. ZARETSKAYA, I. CUTCUTACHE, S. ROSEN, T.L. MADDEN: BMC Bioinformatics, 13, 134 (2012) "Primer-BLAST: A tool to design target-specific primers for polymerase chain reaction") was used to search for homology between the sequences of the candidate primers and the sequences registered with GenBank, to extract primers of high specificity. The extracted primers of high specificity were evaluated for detection sensitivity using the genome DNA of strain D17, and the real-time PCR reaction conditions were optimized for those primers that would permit detection at high sensitivity.

The standards used under the real-time PCR method were produced according to the procedure below. First, the Cica Geneus DNA Extraction Reagent ST (manufactured by Kanto Chemical) was used to extract the genome DNA of strain D17. From the extracted genome DNA, partial sequence of the target genes were amplified using the developed primers, and then refined using NucleoSpin PCR and Gel Clean-up (manufactured by Takara Bio). Next, the refined partial sequences were put through TA cloning using the DynaExpress TA PCR Cloning Kit (pTAC-1) with Jet Competent Cell (manufacture by BioDynamics Laboratory) and, from the resulting clones, those having a plasmid in which a PCR product had been inserted were selected and cultured, and, after extracting plasmid using the FastGene Plasmid Mini Kit (manufactured by Nippon Genetics), used as standards. The prepared standards were put through real-time PCR, and the amplification efficiencies and r² values of the calibration curves were calculated.

The PCR primers shown in Table 2 below, targeting nine genes that are considered to be involved in the decomposition of 1,4-dioxane, were designed and the specificity and detection sensitivity of each of them were examined. As a result, the primer set for detecting the *thmC* gene (corresponding to Probe ID 0864 in Example 1) which is presumed to be coding the monooxygenase component MmoB/DmpM, wherein such primer set comprises a primer containing a base sequence set forth by SEQ ID NO: 1 (5'-TGATTATGTGGGGCTGGTTATG-3') and a primer containing a base sequence set forth by SEQ ID NO: 2 (5'-CGAGGAAAGTTGTGTTCGTGATG-3'), was able to detect strain D17 with the highest specificity and sensitivity.

**[Table 2]**

| Probe ID | Estimated protein | Name of gene |
|---|---|---|
| 0863 | Methane/phenol/toluene hydroxylase | *thmB* |
| 0864 | Monooxygenase component MmoB/DmpM | *thmC* |
| 1673 | glyoxylate carboligase | |
| 2838 | glyoxylate reductas | |
| 3262 | glyoxalase/bleomycin resistance protein/dioxygenase | |
| 5488 | methane/phenol/toluene hydroxylase | *thmB* |
| 5884 | Ferredoxin--NAD(+) reductase | *thmD* |
| 7547 | Regulatory protein ArsR | |
| 7546 | glyoxalase/bleomycin resistance protein/dioxygenase | |

It should be noted that, as mentioned above, strain D17 has four *thmC* genes (Probe IDs 0864, 3016, 5490, 6263) on its genome. Table 3 shows the homologies (%) of the sequences of each pair of these *thmC* genes. The designed primers target the *thmC* gene corresponding to Probe ID 0864, so the other *thmC* genes are not detected because they have different gene sequences.

**[Table 3]**

| | thmC_3016 | thmC_6263 | thmC_0864 | thmC_5490 |
|---|---|---|---|---|
| thmC_3016 | 100 | 52.3 | 45.4 | 47.2 |
| thmC_6263 | 52.3 | 100 | 52.8 | 44.9 |
| thmC_0864 | 45.4 | 52.8 | 100 | 39.8 |
| thmC_5490 | 47.2 | 44.9 | 39.8 | 100 |

It should be noted that *Pseudonocardia* sp. strain ENV458, *Rhodococcus* sp. strain YYL and *Pseudonocardia* sp. strain K1, which are THF-degrading bacteria, as well as strain CB1190 which is a 1,4-dioxane-degrading bacterium, also have thm genes of high homology and therefore these bacteria may also be detectable using the designed PCR primers.

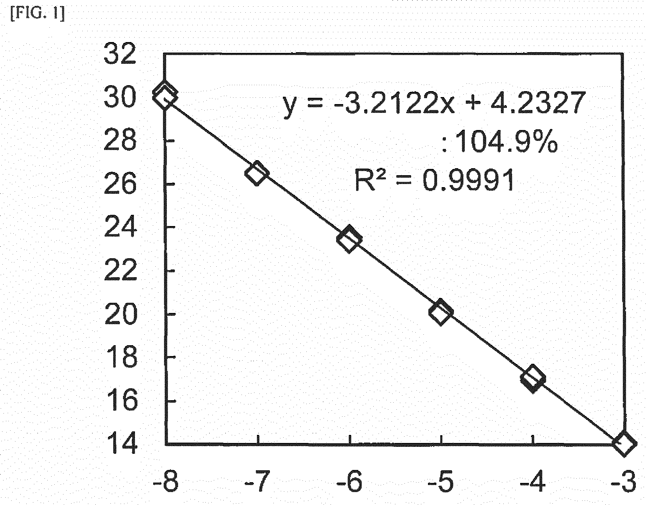

Also, when experimental examination was conducted regarding the conditions for the real-time PCR method that uses the designed PCR primers, it was made clear that quantification with the highest specificity and sensitivity was possible under the conditions shown in Table 4 below. Accordingly, standards for use in quantitative analysis according to real-time PCR method (PCR-amplified products obtained from the genome of strain D17, in which plasmid pTAC-1 was inserted) were prepared, and examined for amplification efficiency, etc. As a result, the amplification efficiencies ranged from 95.3% to 104.9%, the calibration curves had a dynamic range of 6 orders, and the coefficients of correlation r2 varied from 0.9937 to 0.9991 (shown in FIG. 1), which is judged sufficient as the performance of a quantitative monitoring tool.

**[Table 4]**

| Target gene | *thmC* gene (NODE_28_length_17400_cov_346.055328_154 [9758 - 9375] (reverse sense)) |
|---|---|
| Forward primer sequence | 5'-TGATTATGTGGGGCTGGTTATG-3' |
| Reverse primer sequence | 5'-CGAGGAAAGTTGTGTTCGTGATG-3' |
| Amplified fragment length | 107 bp |
| Real-time PCR reaction conditions | 95°C for 1 min x 1 cycle {95°C for 30 sec, 62°C for 30 sec, 72°C for 30 sec} x 32 cycles Melting curve prepared at 60 to 95°C |

### Example 3

The relationship between the quantified value of strain D17 according to the real-time PCR method using a primer set comprising a primer that contains a base sequence set forth by SEQ ID NO: 1 (5'-TGATTATGTGGGGCTGGTTATG-3') and a primer, as a reverse primer, that contains a base sequence set forth by SEQ ID NO: 2 (5'-CGAGGAAAGTTGTGTTCGTGATG-3') on one hand, and the turbidity (the measured result OD₆₀₀) and the quantified value according to the direct counting method using acridine orange (AODC) on the other, was investigated.

Strain D17 was cultured in an MGY medium (Malt Extract: 10 g/L, glucose: 4 g/L, Yeast Extract: 4 g/L) for two weeks. Next, bacteria cells were collected by means of centrifugal separation and then washed twice using saline solution, to prepare bacterial cell solutions of different turbidities (OD₆₀₀), and the prepared bacterial cell solutions were subjected to the AODC method and the real-time PCR method. It should be noted that the bacterial cell solutions had OD₆₀₀ numbers of 0.00015, 0.0089, 0.1058, 1.01305 and 10.01145, respectively.

### <AODC>

2.29 mL of dye preservation solution (1.75% of NaCl, 0.005% of acridine orange, 0.87% of glutaraldehyde) and 0.01 mL of each bacterial cell solution were mixed and reacted for 5 minutes. This reacted mixed solution was suction-filtered using the Nuclepore membrane filter (made of polycarbonate, black type, 0.2 µm) and the filtrate residue was washed using distilled water, and then observed with a fluorescent microscope to count the number of bacterial cells. It should be noted that, if the bacterial cell solution had high turbidity, it was diluted using saline solution as deemed appropriate, before being mixed with the dye preservation solution.

### <Real-Time PCR Method>

2 mL of each bacterial cell solution was centrifugally separated (8500 x g, 4°C, 5 minutes) and the supernatant was removed, after which 100 µL of the Cica Geneus DNA Extraction Reagent ST (manufactured by Kanto Chemical) was added to and mixed with the remaining liquid and the mixture was incubated for 20 minutes at 65°C and 3 minutes at 94°C. The resulting liquid was centrifugally separated (20400 x g, 4°C, 5 minutes), and the supernatant was used as a DNA template. Real-time PCR was implemented with 20 µL of a reaction system (10 µL of GeneAce SYBR qPCR Mix α (Nippon Gene), 0.5 µM of forward primer, 0.5 µM of reverse primer, 2 µL of DNA template), using the reaction conditions in Table 4. Then, the concentration of strain D17 in the bacterial cell solution was obtained as the gene copy concentration, based on the quantified value according to the real-time PCR method.

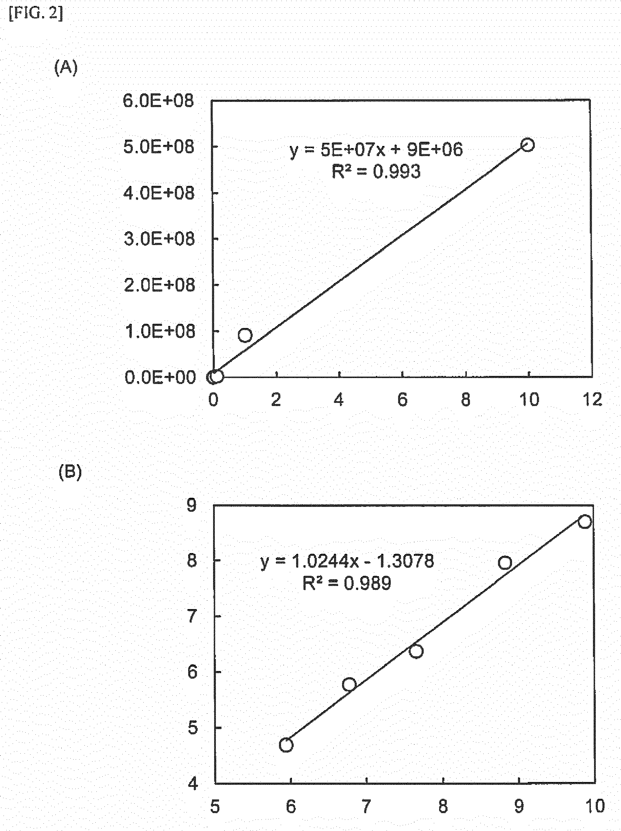

FIG. 2 (A) shows the correlation between the quantified value according to the real-time PCR method and the measured result of turbidity, while FIG. 2 (B) shows the correlation between the quantified value according to the real-time PCR method and the quantified value according to the direct counting method. The quantified value according to the real-time PCR method had high correlation with both the turbidity and the quantified value according to the direct counting method, confirming the utility of the real-time PCR method as a quantitative monitoring method. While the quantified value according to the real-time PCR method is lower, by one order, than the quantified value according to the direct counting method, this is probably due to the effects of DNA extraction efficiency, etc. It should be noted that, because the DNA template preparation method used was found, in the preliminary examination, to be the method associated with the highest DNA extraction efficiency, it is not easy to further improve the quantified value according to the real-time PCR method. For this reason, a preferable method is to convert the result to bacteria cell concentration based on the correlation between the quantified value according to the real-time PCR method and the quantified value according to the direct counting method, as shown in FIG. 2 (B).

### [Sequence Listing]

PTC_1,4-dioxane_20160315_175731_5.txt

## Claims

1. A primer set comprising a primer that contains a base sequence set forth by SEQ 1D NO: 1 and a primer that contains a base sequence set forth by SEQ ID NO: 2.

2. A method for detecting 1,4-dioxane-degrading bacteria, comprising: performing a PCR method using a primer set according to Claim 1, and determining, based on whether or not gene amplification has occurred, whether or not microorganisms having a *thmC* gene are present.

3. A method for quantifying 1,4-dioxane-degrading bacteria, **characterized by** quantifying microorganisms having a *thmC* gene, based on an amplified amount of *thmC* genes obtained by a real-time PCR method that uses a primer set according to Claim 1.


SEQUENCE LISTING

<110>  TAISEI CORPORATION
       OSAKA UNIVERSITY
       SCHOOL JURIDICAL PERSON KITASATO INSTITUTE

<120>  Primers for 1,4-dioxane-decomposing bacterium, and method of
detection and quantification for 1,4-dioxane-decomposing bacterium.

<130>  NATC160624FU

<150>  JP 2015-126202
<151>  2015-06-24

<160>  2

<170>  PatentIn version 3.5

<210>  1
<211>  22
<212>  DNA
<213>  Artificial Sequence

<220>
<223>  a sequence of forward primer

<400>  1
tgattatgtg gggctggtta tg                                              22


<210>  2
<211>  23
<212>  DNA
<213>  Artificial Sequence

<220>
<223>  a sequence of reverse primer

<400>  2
cgaggaaagt tgtgttcgtg atg                                             23